# FASCICULE DE BREVET EUROPEEN

(11) **EP 3 229 923 B1**
(45) Date de publication et mention de la délivrance du brevet: **29.01.2020**
(21) Numéro de dépôt: 15820274.7
(22) Date de dépôt: 08.12.2015
(51) Int. Cl.: A61Q 19/08, A61K 8/97

(54) **UTILISATION COSMÉTIQUE D'UN EXTRAIT D'ESCHSCHOLTZIA CALIFORNICA**
KOSMETISCHE VERWENDUNG EINES EXTRAKTS AUS ESCHSCHOLTZIA CALIFORNICA
COSMETIC USE OF AN EXTRACT OF ESCHSCHOLTZIA CALIFORNICA

(30) Priorité: 09.12.2014 FR 1402796
(43) Date de publication de la demande: 18.10.2017
(73) Titulaire: Laboratoires Clarins, 75823 Paris Cedex 17 (FR)
(72) Inventeur: COURTIN, Olivier, 92200 Neuilly-sur-Seine (FR); WEBER, Sandrine, 95830 Cormeille-en-Vexin (FR)
(74) Mandataire: Gevers & Orès
(86) Numéro de dépôt international: PCT/IB2015/059443
(87) Numéro de publication internationale: WO 2016/092469

(56) Documents cités:
- FR-A1- 2 555 443
- DWECK A C: "ISOFLAVONES, PHYTOHORMONES AND PHYTOSTEROLS", JOURNAL OF APPLIED COSMETOLOGY, INTERNATIONAL EDIEMME, ROME, IT, vol. 24, no. 1, 1 janvier 2006 (2006-01-01), pages 17-32, XP009082606, ISSN: 0392-8543
- DATABASE GNPD [Online] MINTEL; mars 2014 (2014-03), "Plush Night Cream", XP002742522, Database accession no. 2320935

## Description

La présente invention concerne l'utilisation cosmétique d'un extrait d'Eschscholtzia Californica ou d'une composition cosmétique comprenant ledit extrait, en particulier pour retarder ou pour lutter contre le vieillissement de la peau et/ou l'apparition des signes de vieillissement de la peau. La présente invention concerne aussi l'utilisation de cet extrait ou de cette composition comme agent anti oxydant; ainsi que pour induire la synthèse de collagène.

La peau est un organe particulier du corps humain. De fine épaisseur, elle est très étendue puisqu'elle recouvre toute la surface du corps et totalise chez l'adulte une surface d'environ 1,6m². Elle a pour fonction de protéger les tissus profonds du milieu extérieur, tant au niveau de la pénétration physique de corps étrangers, que de l'immunité, de la régulation de la température, et de la déperdition de fluides. Enfin, les contraintes mécaniques auxquelles elle est soumise en permanence lui imposent, plus que tout autre organe, une structure solide et cohérente associée à une grande souplesse. Malgré quelques différences d'aspect suivant le site anatomique, elle présente toujours la même structure morphologique de base. Elle est constituée à sa surface de l'épiderme, en profondeur du derme, et de la jonction dermo-épidermique qui les sépare et les relie.

Le rôle du derme est de maintenir la souplesse et la résistance de la peau. C'est un tissu conjonctif dense, fibreux et élastique reposant sur une couche graisseuse appelée hypoderme, et au sein duquel baignent des cellules mésenchymateuses, les fibroblastes. Ceux-ci sont responsables de la synthèse des constituants de la matrice extracellulaire qui les entoure, véritable tissu de soutien de la peau. La matrice extracellulaire est structurée par une trame fibreuse faite de faisceau de collagène et de fibres élastiques (élastine et fibrilline), en vagues parallèles à la surface de la peau.

Au-delà de son rôle physique de soutien de l'épiderme, le derme assure d'autres fonctions essentielles. Sa vascularisation importante lui permet de réaliser des échanges nutritifs avec l'épiderme. Elle lui permet aussi d'être riche en cellules immunitaires de type macrophages et cellules dendritiques, acteurs essentiels de la défense immunitaire de la peau. De plus, c'est un tissu innervé possédant des récepteurs sensitifs responsables du sens du toucher. Enfin, il intervient dans les phénomènes de cicatrisation, et de régulation de la prolifération et de la différenciation de kératinocytes en synthétisant des cytokines et des facteurs de croissance solubles à destination de l'épiderme.

Le collagène confère à la peau sa résistance aux tensions et à la traction. L'élastine permet à la peau de s'étirer et de revenir en place après déformation. Elle confère son élasticité au revêtement cutané. La matrice extracellulaire contient aussi des protéoglycanes et des glycosaminoglycanes, qui forment une substance fondamentale très hydratée, dans laquelle sont noyées les protéines fibreuses. Enfin, des glycoprotéines de structure assurent l'interface entre la matrice et les cellules en liant les intégrines membranaires.

La matrice extracellulaire de la peau est la matière interstitielle qui entoure les structures cutanées et soutient l'épiderme. Au sein du réseau des protéines fibreuses, le collagène est le principal composant insoluble qui contribue aux propriétés mécaniques de la peau. Les « fibres de collagène » du derme sont constituées respectivement de collagène I et de collagène III, autour d'un axe composé de collagène V. Ces collagènes appartiennent au groupe des collagènes fibrillaires. Chez l'adulte, le collagène I est, en moyenne, six fois plus abondant que le collagène III.

Les fibrilles de collagène I ont une énorme résistance à la traction, et peuvent donc être étirées sans être cassées. Ces fibrilles se disposent côte à côte en rangées parallèles, appelées fibres de collagène. Le collagène III se compose de trois chaînes alpha 1 (III) et est prédominant dans la peau fœtale et les structures extensibles. La proportion de collagène I augmente quand on se rapproche de l'hypoderme. La matrice extracellulaire contient également de la fibronectine, de la laminine, de l'élastine ainsi que de la substance fondamentale de protéoglycanes.

Malgré la grande stabilité de ses constituants, la matrice extracellulaire est une structure dynamique produite en majeure partie localement par les cellules du tissu conjonctif. Dans ce contexte, les fibroblastes jouent un rôle essentiel dans l'entretien et la régénération de ce tissu. Leur capacité à synthétiser et à dégrader le collagène et d'autres macromolécules de la matrice extracellulaire, ou encore à organiser ou à orienter les fibres est critique dans l'homéostasie du tissu cutané.

Pendant le vieillissement, certaines anomalies de la communication entre les cellules et la matrice environnante peuvent conduire à une pléthore de modifications qualitatives et quantitatives de la structure de la matrice extracellulaire. Par exemple, Le rapport collagène I sur collagène III diminue au cours du vieillissement.

Un radical libre est une espèce chimique, atome ou molécule, contenant un électron non apparié. Extrêmement instable, ce composé peut réagir avec les molécules les plus stables pour apparier son électron. Il peut soit arracher un électron (se comportant comme un oxydant), soit en céder un (agissant alors comme un réducteur) . Cette première réaction conduit généralement à la formation en chaîne de nouveaux radicaux ; ceci explique que la production d'un premier radical libre puisse causer d'importantes lésions dans une cellule.

L'O₂ est une molécule biradicalaire formée de deux atomes présentant sur leur orbitale externe deux électrons non appariés. Il est donc susceptible de capter facilement un puis deux électrons pour être partiellement réduit en O₂^{*-} puis en H₂O₂. Il est ainsi à l'origine de la formation d'espèces réactives oxygénées (Reactive Oxygen Species : ROS).

L'appellation ROS inclut les radicaux libres de l'oxygène : anion superoxyde (O₂^{·-}), radical hydroxyle (OH^{·}) mais aussi certains dérivés oxygénés non radicalaires dont la toxicité est importante tels que le peroxyde d'hydrogène (H₂O₂).

Les dommages induits par les ROS sont une peroxydation des lipides, une oxydation des protéines, des mutations de l'ADN. Ces altérations peuvent conduire à des pertes de fonction et d'intégrité, voire à la mort cellulaire notamment par l'intermédiaire de l'apoptose (mort cellulaire programmée). Les ROS initient également l'apoptose en activant l'ouverture du pore de transition de perméabilité (mitochondrie).

Le stress oxydant étant principalement d'origine mitochondriale, ces organites sont les premières cibles des ROS.

Les fonctions de la mitochondrie sont donc particulièrement exposées aux dommages oxydatifs provoquant principalement une diminution de la synthèse d'ATP mais aussi engageant la cellule dans un programme de mort cellulaire par apoptose avec l'induction du pore de transition de perméabilité.

Lors du vieillissement de l'organisme en général et de la peau en particulier, il a été mis en évidence une surproduction des espèces réactives à l'oxygène ainsi qu'une diminution des mécanismes de protection antioxydants. Ces phénomènes provoquent alors un déséquilibre, la production de radicaux libres dépassant les capacités de défenses anti-oxydantes. La majoration de la quantité de radicaux libres présente au niveau cutané provoque des dommages à plusieurs niveaux. Sont touchés les protéines, les membranes cellulaires et l'ADN. Des études ont montré une augmentation de la lipopéroxydation avec l'âge affectant l'intégrité des membranes cellulaires puisqu'elles sont riches en lipides. Ces altérations modifient les fonctions des cellules cutanées ; par exemple, les fibroblastes des peaux âgées sont plus vulnérables à l'accumulation de protéines oxydées résultant d'un stress oxydant et perdent peu à peu leurs fonctions de synthèse des constituants de la matrice extracellulaire dermique.

La demanderesse a mis en évidence de manière surprenante qu'un extrait d'Eschscholtzia Californica ou qu'une composition comprenant un tel extrait protège du vieillissement cutané en favorisant la synthèse de collagène, c'est-à-dire en induisant son expression résultant en une teneur accrue de cette protéine, et en protégeant les cellules cutanées du stress oxydatif. On peut donc utiliser un extrait d'Eschscholtzia Californica comme agent actif anti vieillissement cutané, comme agent actif induisant l'expression du collagène et/ou comme agent anti oxydant.

Eschscholtzia Californica, aussi appelée pavot de Californie ou escholtzia, est une plante herbacée annuelle de la famille des papavéracées, haute de 30 à 40cm. Les fleurs jaunes, orangées de 3 à 5cm de diamètre possèdent une corolle à 4 pétales. Le fruit est une longue capsule riche d'une multitude de graines.

Cette plante est utilisée traditionnellement afin de réduire les insomnies et de réguler le sommeil, traiter l'anxiété ou encore pour calmer l'agitation des enfants.

Elle est connue pour contenir des isoflavones (Dweck A.C. « Isoflavones, Phytohormones and phytosterols », Journal of Applied Cosmetology, International Ediemme, Rome, IT, vol. 24, n°1, 1 janvier 2006, pages 17-32). Bien que certaines isoflavones, de par leur activité oestrogénique, soient décrites par Dweck comme pouvant avoir un effet anti-âge sur la peau, Dweck souligne que les isoflavones portant un groupement methoxy, comme celles présentes chez Eschscholtzia californica, ne sont pas capables de se lier efficacement aux récepteurs aux oestrogènes et, en conséquence, d'induire un effet oestrogénique. Les activités de cette plante observées par la Demanderesse sont donc tout à fait inattendues.

L'utilisation cosmétique selon la présente invention met préférentiellement en œuvre un extrait d'Eschscholtzia Californica ou une composition cosmétique comprenant un tel extrait.

L'extrait est préférentiellement un extrait des parties aériennes d'Eschscholtzia Californica et en particulier un extrait des parties aériennes fleuries. De façon avantageuse, cet extrait est un extrait hydro-alcoolique glycériné. De préférence, il s'agit d'un extrait hydro-alcoolique glycériné obtenu selon un procédé comprenant au moins les étapes suivantes :
- séchage des parties aériennes fleuries ;
- extraction - macération à froid avec alcool (15 à 20%), glycérine (14 à 16%) et eau (QSP 100), à température ambiante pendant 10 jours avec une agitation manuelle quotidienne à une température de 19°C environ;
- pressage ;
- décantation puis filtration.

L'extrait d'Eschscholtzia Californica utilisé selon l'invention est un liquide brun à rouge d'odeur aromatique. Il présente les caractéristiques analytiques suivantes :
Densité relative : 1 - 1,035
Indice de réfraction : 1,355 -1,375
Résidu sec : 12,00 minimum

On peut se procurer un extrait d'Eschscholtzia Californica selon l'invention auprès du fournisseur de matières premières Fytosan sous la dénommination « extrait hydroalcoolique glycériné Bio ».

De préférence la composition cosmétique selon l'invention comprend de 0,01 à 10% d'extrait d'Eschscholtzia Californica en poids de matière sèche par rapport au poids total de la composition. Avantageusement, la composition comprend de 0,01 à 5% d'extrait d'Eschscholtzia Californica en poids de matière sèche par rapport au poids total de la composition.

Les compositions selon l'invention peuvent encore comprendre un ou plusieurs agents de formulation ou additifs d'usage connu et classique dans les compositions cosmétiques et dermatologiques tels que, à titre d'exemples et de façon non limitative, des adoucissants, des colorants, des actifs filmogènes, des tensioactifs, des parfums, des conservateurs, des émulsionnants, des huiles, des glycols, des vitamines comme la vitamine E, des filtres UV, etc... Grâce à ses connaissances en matière de cosmétique, l'homme du métier saura quels agents de formulation ajouter aux compositions de l'invention et en quelles quantités en fonction des propriétés recherchées.

Les compositions selon l'invention peuvent se présenter sous toute forme connue de l'homme du métier dans le domaine de la cosmétologie et de la dermatologie sans autre restriction galénique que l'application sur le visage et sur le corps. De façon avantageuse, les compositions selon l'invention se présentent sous la forme d'un gel, d'une crème, d'une lotion, d'une huile, d'un lait, d'un spray, etc...

La demanderesse a démontré que l'extrait d'Eschscholtzia Californica utilisé selon l'invention ainsi que la composition cosmétique le comprenant peuvent être utilisés par application sur la peau pour favoriser la synthèse de collagène, par exemple, par induction de son expression ; ou encore comme agent anti oxydant pour protéger les cellules cutanées du stress oxydatif.

En conséquence l'extrait d'Eschscholtzia Californica ou la composition cosmétique le comprenant selon l'invention peuvent être utilisés pour prévenir, pour retarder, pour lutter contre, pour traiter ou réduire le vieillissement de la peau et/ou l'apparition des signes de vieillissement de la peau.

On entend par signes de vieillissement de la peau : les rides, les ridules, le relâchement cutané, la perte d'élasticité des fibres cutanées, une peau flétrie, une peau amincie, et une peau terne et/ou sans éclat.

Grâce son action sur l'induction de la synthèse du collagène dans la peau et ses propriétés anti-oxydantes, selon une variante préférée de l'invention, l'extrait d'Eschscholtzia Californica peut être utilisé pour réparer les manifestations cutanées du vieillissement ; en particulier estomper les rides et les ridules, ou encore redensifier une peau flétrie ou amincie.

L'extrait d'Eschscholtzia Californica utilisé selon l'invention et la composition cosmétique le comprenant peuvent également être utilisés pour redensifier et/ou raffermir et/ou tonifier la peau.

L'extrait d'Eschscholtzia Californica selon l'invention et la composition cosmétique le comprenant sont utilisés par application sur la peau ; il peut s'agir indifféremment de la peau du visage ou du corps.

Lorsque qu'il s'agit de la peau du visage, c'est préférentiellement la peau de la face, du cou, du décolleté et/ou du contour des yeux.

Selon une variante particulière, L'extrait d'Eschscholtzia Californica selon l'invention et la composition cosmétique le comprenant sont appliqués sur la peau du corps pour la raffermir et/ou la tonifier, par exemple dans le cadre d'un amincissement.

Les exemples ci-après concernent, d'une part l'évaluation de l'effet d'un extrait d'Eschscholtzia Californica sur la synthèse de collagène I, et sur son effet anti oxydant et d'autre part des compositions objets de la présente invention.

Les exemples font référence aux figures suivantes dans lesquelles :

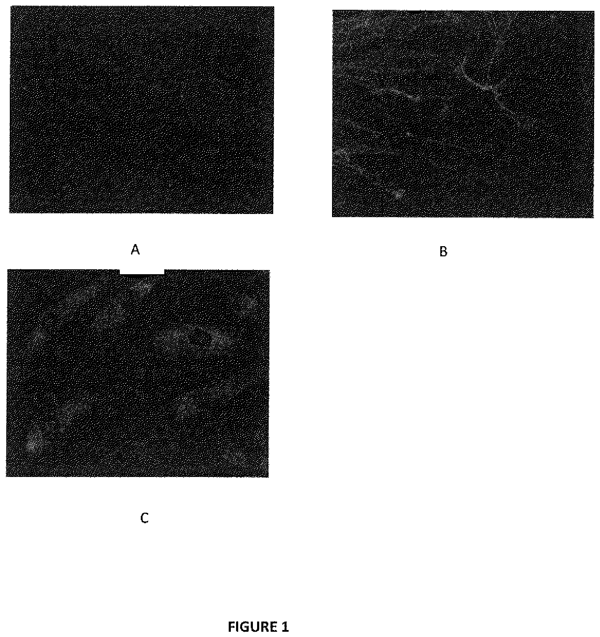

- La figure 1 représente l' immunofluorescence du collagène I (TRITC) pour le témoin (photo A), le témoin positif la vitamine C (photo B) et pour une concentration d'extrait d'Eschscholtzia Californica à 0,05 % (photo C), (Observations en microscopie optique à fluorescence grossissement x200).

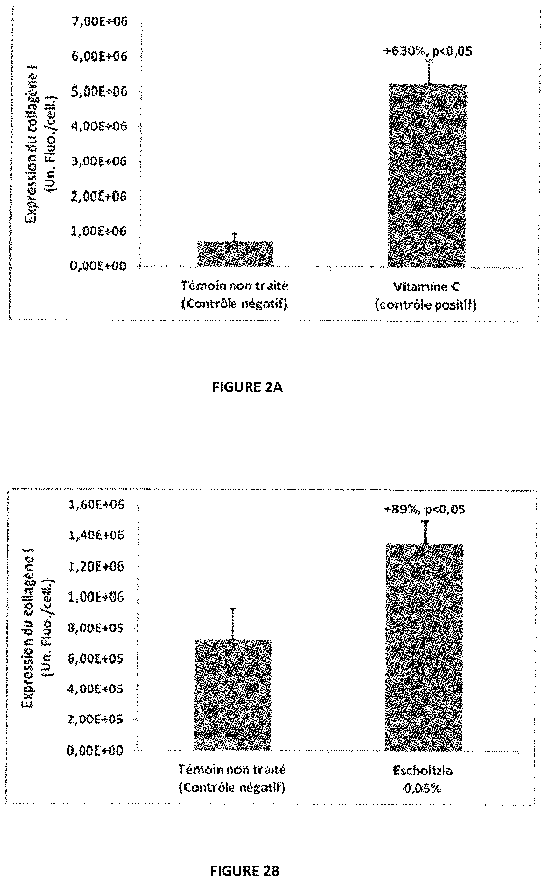

- Les figures 2A et 2B représentent l'expression du collagène I exprimée en unité de fluorescence par noyau par des fibroblastes en présence ou non de vitamine C et d'un extrait d'Eschscholtzia Californica à 0,05%.

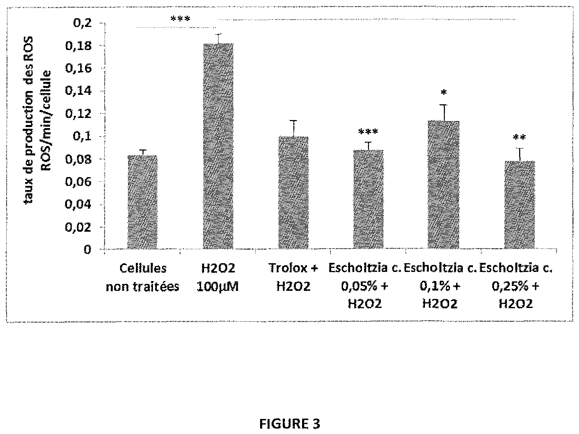

- La figure 3 représente le taux de production des ROS par minute et par cellule induit par H₂O₂ à 100µM par des fibroblastes humains en présence ou non de trolox et d'extrait d'Eschscholtzia Californica à différentes concentrations.

### I. EVALUATION DE L'ACTIVITE D'UN EXTRAIT D'ESCHSCHOLTZIA CALIFORNICA SUR LA SECRETION DU COLLAGENE DE TYPE I

### A.MATERIEL ET METHODE

Les fibroblastes sont ensemencés dans des plaques 6 puits avec lamelle de verre à raison de 70.000 cellules par boîte dans du milieu MEM (minimum Essential Médium) . 48h après, les cellules sont traitées ou non (témoin) par l'extrait de Eschscholtzia californica à 0,05%, 0,1% et 0,25% ou par un contrôle positif (vitamine C) et incubées à l'étuve pendant 72 heures à 37°C, 5% CO₂.

Chaque tapis cellulaire est ensuite rincé, fixé au méthanol (-20°C) avant de procéder à la révélation du collagène de type I.

Les cellules sont incubées avec le 1^{er} anticorps dirigé contre la protéine d'intérêt puis avec l'anticorps secondaire dirigé contre le 1^{er} anticorps couplé au fluorochrome TRITC (Tetra methyl Rhodamine Iso Thio Cyanate, dérivé de la Rhodamine). Le temps d'incubation entre chaque anticorps est d'1 heure. Les noyaux cellulaires sont révélés par un marquage au Dapi (Di Aminido Phenyl Indol).

Les lamelles sont montées sur lame de verre puis observées au microscope à fluorescence (Nikon Eclipse 50i). Plusieurs champs de la population cellulaire sont pris en photo. Les images de fluorescence TRITC (en rouge) indiquent les régions de localisation de la protéine d'intérêt. Les clichés photographiques sont analysés par le logiciel d'analyse d'image NIS Elements (Nikon).

### B. RESULTATS

Cette étude démontre que l'extrait de Eschscholtzia californica à 0.05% induit de manière significative la synthèse du collagène de type I par les fibroblastes, conduisant à une augmentation de la teneur en collagène dans les puits traités avec l'extrait d'Eschscholtzia californica. L'extrait d'Eschscholtzia californica stimule la synthèse matricielle du derme par les fibroblastes. Ces propriétés confèrent à cet extrait un rôle anti-âge.

### II. EVALUATION DE L'ACTIVITE D'UN EXTRAIT D'ESCHSCHOLTZIA CALIFORNICA COMME ANTI OXYDANT

La méthodologie consiste en la quantification de l'activité antioxydante de l'extrait d'Eschscholtzia californica vers les ROS à l'aide un marqueur fluorescent.

### A. MATERIEL ET METHODE

Les cellules sont pré-incubées avec la sonde fluorescente à 37°C pendant 30 minutes. Après lavage, les cellules sont co-incubées avec le composé de référence antioxydant, Trolox 10 µM ou l'extrait de Escholtzia californica à des concentrations déterminées non cytotoxiques en présence du composé de référence pro-oxydant, l'H₂O₂ à 100µM. La production de ROS cellulaires est immédiatement enregistrée pendant 60 minutes. La fluorescence est enregistrée toutes les cinq minutes en utilisant un lecteur de microplaques (Varioskan-Thermo). Les cellules sont maintenues à 37°C au cours de la cinétique. Tous les composés sont testés en quadruplicate. Leurs effets sont comparés simultanément aux témoins négatif (H₂O₂) et positif (trolox).

### B. RESULTATS

Cette étude démontre que l'extrait de Eschscholtzia californica aux doses testées (0,05%, 0,1% et 0,25%) réduit de façon très marquée, quasi totalement dans certains des cas, la production des ROS cellulaires induites par H₂O₂.

### III. EXEMPLES DE FORMULATION

Les pourcentages sont des pourcentages en poids par rapport au poids total de la composition.

**CREME DE JOUR SPF 15**

| | **%** |
|---|---|
| XANTHAN GUM | 0,20 |
| GLYCERIN | 5,00 |
| CAPRYLYL GLYCOL | 0,30 |
| HYDROXYETHYL ACRYLATE/SODIUM ACRYLOYLDIMETHYL TAURATE COPOLYMER | 0,75 |
| POTASSIUM CETYL PHOSPHATE | 0,70 |
| CETEARYL ALCOOL CETEARYL GLUCOSIDE | 1,50 |
| TOCOPHERYL ACETATE | 0,20 |
| C12-15 ALKYL BENZOATE | 4,00 |
| BUTYLOCTYL SALICYLATE | 5,00 |
| HOMOSALATE | 5,00 |
| BUTYL METHOXYDIBENZOYLMETHANE | 3,00 |
| OCTOCRYLENE | 2,70 |
| EXTRAIT DE ESCHSCHOLTZIA CALIFORNICA | 0,50 |
| TOCOPHEROL | 0,05 |
| CONSERVATEURS | Q.S. |
| PARFUM | Q.S. |
| EAU DEMINERALISEE | Q.S.P 100 |

**CREME DE NUIT**

| | **%** |
|---|---|
| GLYCERYL STEARATE / PEG-100 STEARATE | 2,50 |
| ISONONYL ISONONANOATE | 4,50 |
| BEURRE DE KARITE | 2,00 |
| CETEARYL ISONONANOATE | 8,00 |
| CETEARYL ALCOHOL / CETEARYL GLUCOSIDE | 3,50 |
| DIMETHICONE | 3,00 |
| CARBOMER | 0,25 |
| CELLULOSE | 0,50 |
| EXTRAIT DE ESCHSCHOLTZIA CALIFORNICA | 1,00 |
| GLYCOLS | 6,20 |
| TOCOPHERYL ACETATE | 0,20 |
| CONSERVATEURS | 0,60 |
| CHELATANT | 0,10 |
| TROMETHAMINE | Q.S.PH |
| PARFUM | Q.S. |
| EAU DEMINERALISEE | Q.S.P 100 |

## Revendications

1. Utilisation cosmétique d'un extrait d'Eschscholtzia Californica pour induire la synthèse de collagène.

2. Utilisation cosmétique, non-thérapeutique, d'un extrait d'Eschscholtzia Californica comme agent anti oxydant pour protéger les cellules cutanées du stress oxydatif.

3. Utilisation cosmétique d'un extrait d'Eschscholtzia Californica pour retarder, pour lutter contre, pour traiter ou réduire le vieillissement de la peau et/ou l'apparition des signes de vieillissement de la peau.

4. Utilisation selon la revendication 3, dans laquelle les signes de vieillissement de la peau sont sélectionnés parmi les rides, les ridules, le relâchement cutané, la perte d'élasticité des fibres cutanées, une peau flétrie, une peau amincie, et une peau terne et/ou sans éclat.

5. Utilisation cosmétique d'un extrait d'Eschscholtzia Californica pour réparer les manifestations cutanées du vieillissement et/ou estomper les rides et les ridules et/ou redensifier une peau flétrie ou amincie et/ou redensifier et/ou raffermir et/ou tonifier la peau du visage et/ou du corps.

6. Utilisation non-thérapeutique d'une composition cosmétique pour induire la synthèse de collagène et/ou pour protéger les cellules cutanées du stress oxydatif, **caractérisée en ce que** ladite composition comprend un extrait d'Eschscholtzia californica.

7. Utilisation selon l'une quelconque des revendications précédentes, dans laquelle l'extrait d'Eschscholtzia Californica est un extrait des parties aériennes fleuries.

8. Utilisation selon l'une quelconque des revendications précédentes, dans laquelle l'extrait d'Eschscholtzia Californica est un extrait hydro alcoolique glycériné.

9. Utilisation selon l'une quelconque des revendications précédentes, **caractérisée en ce que** ledit extrait est formulé dans une composition cosmétique qui comprend de 0,01 à 10% dudit extrait en poids de matière sèche par rapport au poids total de la composition, de préférence de 0,01 à 5% dudit extrait en poids de matière sèche par rapport au poids total de la composition.

10. Utilisation selon la revendication 9, dans laquelle ladite composition cosmétique comprend en outre un ou plusieurs agents de formulation ou additifs tels que des adoucissants, des colorants, des agents filmogènes, des tensio-actifs, des parfums, des conservateurs, des émulsionnants, des huiles, des glycols, filtres UV, et des vitamines.

## Patentansprüche

1. Kosmetische Verwendung eines Extrakts von Eschscholtzia Californica zum Induzieren der Kollagensynthese.

2. Kosmetische, nicht-therapeutische Verwendung eines Extrakts von Eschscholtzia Californica als Antioxidationsmittel zum Schützen der Hautzellen vor oxidativem Stress.

3. Kosmetische Verwendung eines Extrakts von Eschscholtzia Californica zum Verzögern, zum Bekämpfen, zum Behandeln oder Verringern der Alterung der Haut und/oder der Entstehung von Zeichen einer Alterung der Haut.

4. Verwendung nach Anspruch 3, wobei die Zeichen einer Alterung der Haut ausgewählt sind aus Falten, Fältchen, Hautschlaffheit, Elastizitätsverlust der Hautfasern, einer welken Haut, einer dünner gewordenen Haut und einer matten und/oder glanzlosen Haut.

5. Kosmetische Verwendung eines Extrakts von Eschscholtzia Californica zum Beheben der Hauterscheinungen der Alterung und/oder zum Verschwindenlassen der Falten und der Fältchen und/oder zum Wiederverdichten einer welken oder dünner gewordenen Haut und/oder Wiederverdichten und/oder Straffen und/oder Beleben der Haut des Gesichts und/oder des Körpers.

6. Nicht-therapeutische Verwendung einer kosmetischen Zusammensetzung zum Induzieren der Kollagensynthese und/oder zum Schützen der Hautzellen vor oxidativem Stress, **dadurch gekennzeichnet, dass** die Zusammensetzung einen Extrakt von Eschscholtzia Californica umfasst.

7. Verwendung nach einem der vorstehenden Ansprüche, wobei der Extrakt von Eschscholtzia Californica ein Extrakt der blühenden Oberteile ist.

8. Verwendung nach einem der vorstehenden Ansprüche, wobei der Extrakt von Eschscholtzia Californica ein glycerinierter hydroalkoholischer Extrakt ist.

9. Verwendung nach einem der vorstehenden Ansprüche, **dadurch gekennzeichnet, dass** der Extrakt in einer kosmetische Zusammensetzung formuliert ist, die von 0,01 bis 10 % des Extrakts in Gewicht der Trockenmasse bezogen auf das Gesamtgewicht der Zusammensetzung umfasst, bevorzugt von 0,01 bis 5 % des Extrakts in Gewicht der Trockenmasse bezogen auf das Gesamtgewicht der Zusammensetzung.

10. Verwendung nach Anspruch 9, wobei die kosmetische Zusammensetzung weiter ein oder mehrere Formulierungsmittel oder Zusatzstoffe, wie Weichmacher, Farbstoffe, Filmbildner, Tenside, Parfums, Konservierungsmittel, Emulgatoren, Öle, Glykole, UV-Filter und Vitamine, umfasst.

## Claims

1. Cosmetic use of an Eschscholtzia Californica extract to induce the synthesis of collagen.

2. Cosmetic, non-therapeutic, use of an Eschscholtzia Californica extract as an anti-oxidant agent to protect the skin cells from oxidative stress.

3. Cosmetic use of an Eschscholtzia Californica extract to delay, to fight against, to treat or reduce the ageing of the skin and/or the appearance of signs of ageing of the skin.

4. Use according to claim 3, wherein the signs of ageing of the skin are selected from wrinkles, fine lines, sagging skin, loss of elasticity of skin fibres, shrivelled skin, thinned skin, and dull and/or non-shiny skin.

5. Cosmetic use of an Eschscholtzia Californica extract to repair the skin manifestations of ageing and/or to diminish wrinkles and fine lines and/or re-densify shrivelled or thinned skin and/or re-densify and/or firm-up and/or tone-up the skin of the face and/or of the body.

6. Non-therapeutic use of a cosmetic composition to induce the synthesis of collagen and/or to protect the skin cells from oxidative stress, **characterised in that** said composition comprises an Eschscholtzia Californica extract.

7. Use according to any one of the preceding claims, wherein the Eschscholtzia Californica extract is an extract of flowery aerial parts.

8. Use according to any one of the preceding claims, wherein the Eschscholtzia Californica extract is a glycerine hydro alcohol extract.

9. Use according to any one of the preceding claims, **characterised in that** said extract is formulated in a cosmetic composition which comprises from 0.01 to 10% of said extract by weight of dry matter with respect to the total weight of the composition, preferably from 0.01 to 5% of said extract by weight of dry matter with respect to the total weight of the composition.

10. Use according to claim 9, wherein said cosmetic composition further comprises one or more formulation agents or additives such as softeners, colourants, film-forming agents, surfactants, perfumes, preservatives, emulsifiers, oils, glycols, UV filters, and vitamins.
